# EUROPÄISCHE PATENTANMELDUNG

(11) **EP 4 684 747 A1**
(43) Veröffentlichungstag der Anmeldung: **28.01.2026**
(21) Anmeldenummer: 24190034.9
(22) Anmeldetag: 22.07.2024
(51) Int. Cl.: A61B 18/12, A61B 34/30, A61B 18/14, A61B 18/00

(54) **MEDIZINISCHES ROBOTERSYSTEM**

(71) Anmelder: Erbe Elektromedizin GmbH, 72072 Tübingen (DE)
(72) Erfinder: SELIG, Peter, 72147 Nehren (DE)
(74) Vertreter: Rüger Abel Patentanwälte PartGmbB

(57) **Zusammenfassung**

Die Erfindung betrifft ein medizinisches Robotersystem (10) mit einem wenigstens einen Roboterarm (12) aufweisenden Roboter (11). An dem wenigstens einen Roboterarm (12) ist wenigstens eine Instrumenteneinheit (19) gehalten und mittels des Roboterarms (12) bewegbar und/oder positionierbar. Jede Instrumenteneinheit (19) hat ein medizinisches Instrument (20), vorzugsweise elektrochirurgisches Instrument (21), sowie einen Generator (22), der ein Ausgangssignal (A), insbesondere HF-Ausgangssignal, für das Instrument (20) erzeugt und bereitstellt. Am Roboterarm (12) ist eine Schnittstelle (32) angeordnet, mit der jede am Roboterarm (12) gehaltene Instrumenteneinheit (19) zur Herstellung einer Kommunikationsverbindung elektrisch und/oder optisch gekoppelt werden kann. Die Verbindung kann drahtlos und/oder drahtgebunden sein. Der Roboter (11) bzw. der Roboterarm (12) hat wenigstens eine Generatorsteuereinheit (31), wobei jede Schnittstelle (32) mit einer zugeordneten Generatorsteuereinheit (31) kommunikationsverbunden ist, um ein Generatorsteuersignal (C) von der Generatorsteuereinheit (31) an die wenigstens eine an die Schnittstelle (32) angeschlossene Instrumenteneinheit (19) zu übertragen. Der Generator (22) und die Generatorsteuereinheit (31) sind somit voneinander getrennt, wobei sich der Generator (22) außerhalb des Roboters (11) befindet, während die wenigstens eine Generatorsteuereinheit (31) Bestandteil des Roboters (11) ist und in den Roboter (11) bzw. den Roboterarm (12) integriert sein kann.

## Beschreibung

Die Erfindung betrifft ein medizinisches Robotersystem. Mittels dem Robotersystem kann eine Bedienperson, beispielsweise ein Operateur, einen menschlichen oder tierischen Patienten behandeln. Das Robotersystem hat einen Roboter mit wenigstens einem Roboterarm. Jeder vorhandene Roboterarm kann ein medizinisches Instrument oder auch mehrere medizinische Instrumente halten. Mittels des wenigstens einen Roboterarms kann ein verwendetes medizinisches Instrument positioniert und bewegt werden, beispielsweise während der Behandlung von biologischem Gewebe des Patienten.

Aus DE 10 2013 002 832 A1 ist ein Roboter für die HF-Chirurgie bekannt. Der Roboter hat wenigstens einen Roboterarm und eine mechanische Schnittstelle zum lösbaren Befestigen eines chirurgischen Instruments. Zusätzlich ist eine HF-Schnittstelle zur Übertragung eines HF-Stroms an das chirurgische Instrument vorhanden. Eine zusätzliche elektrische Leitungsverbindung vom Instrument zu einem HF-Generator außerhalb des Roboterarms kann dadurch entfallen. Der HF-Generator ist im Roboterarm integriert und wird von einer Steuereinrichtung des Roboters gesteuert.

US 2016/0270840 A1 betrifft ein elektrochirurgisches Instrument, in dem der Generator innerhalb des Instrumentengehäuses integriert ist.

Aus EP 4 192 379 A1 ist eine modulare Vorrichtung für robotergestützte Elektrochirurgie bekannt. An einem Roboterarm ist ein Instrumentenhalter vorhanden, um ein medizinisches Instrument mit dem Roboterarm zu verbinden. In dem Instrumentenhalter sind Aussparungen vorhanden, in die elektrochirurgische Kapseln eingesetzt werden können. In der Kapsel ist ein Steuermodul, ein Generatormodul und ein Ausgangsmodul vorhanden. Das Ausgangsmodul ist elektrisch mit einem Ausgangsanschluss verbunden. Über den Ausgangsanschluss können elektromagnetische Signale an ein Instrument übermittelt werden, das elektrisch mit dem Ausgangsanschluss verbunden ist.

In EP 3 852 664 A1 ist ein Robotersystem beschrieben, bei dem ein Generator für ein medizinisches Instrument auf dem Roboterarm angeordnet ist.

Ausgehend vom Stand der Technik kann es als Aufgabe der vorliegenden Erfindung angesehen werden, ein verbessertes Robotersystem bereitzustellen, das zum einen eine hohe elektromagnetische Verträglichkeit ermöglicht und zum anderen einen flexiblen und einfachen Einsatz unterschiedlicher medizinischer Instrumente ermöglicht.

Diese Aufgabe wird durch ein medizinisches Robotersystem mit den Merkmalen des Patentanspruches 1 gelöst.

Das medizinische Robotersystem gemäß der vorliegenden Erfindung weist einen Roboter mit wenigstens einem Roboterarm auf. Der Roboterarm ist dazu eingerichtet, eine oder mehrere Instrumenteneinheiten zu halten. Hierzu kann der Roboterarm eine Halteeinrichtung für eine oder für mehrere Instrumenteneinheiten aufweisen. Über die Halteeinrichtung wird eine lösbare Verbindung mit der Instrumenteneinheit hergestellt, beispielsweise eine kraftschlüssige und/oder formschlüssige Verbindung.

Jede Instrumenteneinheit hat ein medizinisches Instrument, beispielsweise ein elektrochirurgisches Instrument. Das Instrument kann ein monopolares oder bipolares Instrument sein. Das Instrument kann für den offenchirurgischen oder laparoskopischen Einsatz eingerichtet sein. Das medizinische Instrument kann auch ein endoskopisches Instrument sein und in Kombination mit einem Endoskop verwendet werden.

Das Instrument ist vorzugsweise ein elektrochirurgisches Instrument. Das Instrument kann eine oder mehrere Instrumentenelektroden aufweisen. Beispielsweise können zwei vorhandene Instrumentenelektroden als Koagulationselektroden verwendet werden. Zusätzlich oder alternativ kann das Instrument eine Schneidelektrode aufweisen. Die vorhandenen Instrumentenelektroden können abhängig von einem angeforderten Betriebszustand des Instruments auch unterschiedliche Funktionen haben. Beispielsweise kann eine Koagulationselektrode beim Schneiden mit einer Schneidelektrode als Neutralelektrode verwendet werden.

Jede Instrumenteneinheit hat außerdem einen Generator. Der Generator ist elektrisch mit dem medizinischen Instrument verbunden. Der Generator kann Bestandteil des Instruments sein und beispielsweise im Instrumentengehäuse angeordnet sein. Der Generator kann auch eine separate Komponente sein, die mechanisch lösbar oder unlösbar mit dem medizinischen Instrument verbunden ist. In diesem Fall sind der Generator und das Instrument elektrisch und mechanisch miteinander verbunden, wobei die Verbindung beispielsweise eine Steckverbindung, Rastverbindung oder eine andere formschlüssige und/oder kraftschlüssige Verbindung aufweisen kann.

Der Generator ist dazu eingerichtet, ein mit einer Frequenz alternierendes elektrisches Ausgangssignal für das zugeordnete Instrument der Instrumenteneinheit bereitzustellen. Das Ausgangssignal kann eine Wechselspannung und/oder ein Wechselstrom sein. Das Ausgangssignal ist insbesondere ein Hochfrequenzsignal mit einer Frequenz in einem Bereich von 100 kHz bis einschließlich 5 MHz und vorzugsweise von 300 kHz bis einschließlich 600 kHz. Mittels des Generators lassen sich weitere Parameter einstellen, beispielsweise ein Scheitelfaktor des Ausgangssignals (englisch: "crest factor") und/oder eine Wellenform des Ausgangssignals (englisch: "waveform") und/oder wenigstens ein Modulationsparameter eines modulierten Ausgangssignals (beispielsweise ein Tastgrad eines pulsweitenmodulierten Ausgangssignals).

Der Roboter hat eine Generatorsteuereinheit. Es können auch mehrere Generatorsteuereinheiten vorhanden sein. Jede Generatorsteuereinheit kann einem einzigen Roboterarm oder mehreren Roboterarmen zugeordnet sein. Wenn ein Roboterarm mehrere Instrumenteneinheiten halten kann, können optional auch mehrere Generatorsteuereinheiten für diesen Roboterarm vorhanden sein.

Jede Generatorsteuereinheit ist dazu eingerichtet, wenigstens einen oder genau einen zugeordneten Generator zu steuern. Hierzu ist die Generatorsteuereinheit mit einer am Roboterarm angeordneten Schnittstelle verbunden. Die Schnittstelle befindet sich vorzugsweise in der Nähe der Instrumentenhalteeinrichtung für die wenigstens eine Instrumenteneinheit. Die Verbindung zwischen der Generatorsteuerung und der Schnittstelle kann mittels einer elektrischen Leitung erfolgen, die im oder am Roboterarm angeordnet ist. Die Schnittstelle hat einen Steueranschluss oder mehrere Steueranschlüsse, wobei an jedem Steueranschluss jeweils ein Generator einer Instrumenteneinheit elektrisch anschließbar ist. Bei hergestellter elektrischer Verbindung zwischen dem Generator und einem Steueranschluss kann ein von der Generatorsteuereinheit erzeugtes Generatorsteuersignal an den angeschlossenen Generator übertragen werden.

Aufgrund der Trennung zwischen wenigstens einen Generatorsteuereinheit des Roboters von dem wenigstens einen Generator ist eine hohe Flexibilität beim Einsatz unterschiedlicher Instrumente erreicht. Jede Instrumenteneinheit hat einen eigenen Generator, der für das zugeordnete Instrument (beispielsweise das elektrochirurgische Instrument) dieser Instrumenteneinheit eingerichtet ist. Der Generator kann sehr einfach mittelbar durch Anordnen des Instruments am Roboterarm gehalten werden. Aufgrund der Anordnung des Generators unmittelbar benachbart zu dem Instrument, für die er das elektrische Ausgangssignal bereitstellt, können elektromagnetische Störungen klein gehalten werden, was wiederum zu einer sehr guten elektromagnetischen Verträglichkeit (EMV) des Robotersystems beiträgt. Das Ausgangssignal, das insbesondere ein Hochfrequenz-Ausgangssignal ist, muss lediglich über eine kurze Strecke (d.h. kurze Länge einer elektrischen Leitung) vom Generator zum Instrument bzw. zu wenigstens einer Instrumentenelektrode des Instruments übertragen werden. Sollten elektromagnetische Abschirmungen erforderlich sein, ist der Aufwand hierfür gering.

Über die Schnittstelle am Roboterarm können sehr einfach anwendungsspezifisch eine oder mehrere benötigte Instrumenteneinheiten mit der Generatorsteuereinheit des Roboters verbunden werden. Ein Auswechseln von Roboterkomponenten ist nicht erforderlich.

Eine einzige Generatorsteuereinheit des Roboters ist prinzipiell ausreichend. In jedem Fall kann die Anzahl von Instrumenteneinheiten und mithin die Anzahl von Generatoren größer sein als die Anzahl der vorhandenen Generatorsteuereinheiten. Dadurch kann der steuerungstechnische Bauteilaufwand gering sein.

Bei der Verwendung eines bipolaren Instruments im Robotersystem wird ein Strom von einer Elektrode in das zu behandelnde Gewebe und von dort über eine weitere Instrumentenelektrode zurück zum Generator geführt. Handelt es sich bei dem Instrument um ein monopolares Instrument, wird ein mittels einer Instrumentenelektrode in das biologische Gewebe eingeleiteter Strom über eine am Patienten angebrachte Neutralelektrode zum Generator zurückgeführt. Hierzu kann am Generator und/oder an der Schnittstelle des Roboterarms ein Neutralelektrodenanschluss für eine Neutralelektrode vorhanden sein. Die Neutralelektrode kann mittelbar über die Schnittstelle am Roboterarm oder alternativ unmittelbar mit mehreren Generatoren unterschiedlicher Instrumenteneinheiten elektrisch verbunden sein.

Es ist vorteilhaft, wenn die Schnittstelle wenigstens einen Identifikationsanschluss aufweist. Mittels des Identifikationsanschlusses kann ein Identifikationssignal an die zugeordnete Generatorsteuereinheit übertragen werden. Das Identifikationssignal ermöglicht eine Identifikation eines Instruments bzw. einer Instrumenteneinheit, die an diesem Roboterarm angeordnet ist, indem es zum Beispiel eine der Instrumenteneinheit zugeordnete Kennung beschreibt oder aufweist. Die Kennung ist vorzugsweise unveränderlich. Die Kennung kann in einem Datenspeicher der Instrumenteneinheit abgespeichert sein. Anhand des empfangenen Identifikationssignals kann die Generatorsteuereinheit feststellen, welche Art bzw. welchen Typ von medizinischem Instrument am Roboterarm vorhanden ist und daraufhin eine entsprechende Steuerung der Instrumenteneinheit über den Steueranschluss der Schnittstelle veranlassen. Die Art bzw. der Typ eines Instruments kann durch eine Eigenschaft oder mehrere beliebige der folgenden Eigenschaften gekennzeichnet sein:
- die Anzahl der vorhandenen Elektroden,
- die Ausführung als monopolares oder bipolares Instrument,
- ein Instrument, das ein Fluid (Gas und/oder Flüssigkeit) zur Gewebebeeinflussung benötigt und/oder verwendet,
- die wenigstens eine Anwendung des Instruments (Schneiden, Koagulieren, Fusionieren, etc.).

Die Instrumenteneinheit kann drahtlos oder drahtgebunden mit dem Identifikationsanschluss der Schnittstelle verbunden sein. Die Verbindung kann elektrisch und/oder elektromagnetisch und/oder optisch hergestellt sein.

Beispielsweise kann bei einem Ausführungsbeispiel eine Erfassungseinheit vorhanden sein, die mit dem Identifikationsanschluss verbunden oder verbindbar ist und die dazu eingerichtet ist, eine Kennung der zugeordneten Instrumenteneinheit bzw. des zugeordneten Instruments zu erfassen. Über die Kennung kann die Generatorsteuereinheit feststellen, um was für einen Instrumententyp es sich handelt und wie dieser Instrumententyp zu steuern ist.

Die Erfassungseinheit kann die Kennung drahtlos oder drahtgebunden erfassen, wobei die Verbindung elektrisch, elektromagnetisch oder optisch hergestellt werden kann. Beispielsweise kann die Erfassungseinheit eine Nahfeldkommunikation (NFC) oder eine RFID-Verbindung (Radio-Frequency Identification) verwenden, um die Kennung zu detektieren. Die Erfassungseinrichtung kann im Generator der Instrumenteneinheit und/oder in der Schnittstelle des Roboterarms angeordnet sein. Zusätzlich oder alternativ kann die Kennung optisch erfassbarer Code sein, beispielsweise ein zweidimensionaler Code (Barcode, QR-Code, usw.)

Es ist außerdem vorteilhaft, wenn die Schnittstelle wenigstens einen Energieversorgungsanschluss aufweist. Der Energieversorgungsanschluss ist mit einer Energiequelle des Roboters bzw. des Robotersystems verbunden, wobei die Energiequelle insbesondere eine Gleichspannung und/oder einen Gleichstrom bereitstellt. Dabei kann entweder die Gleichspannung oder der Gleichstrom eingeprägt sein.

Es ist bevorzugt, wenn die Schnittstelle wenigstens einen Rückmeldungsanschluss aufweist. Jeder Rückmeldungsanschluss kann mit einem Generator einer an diesem Roboterarm gehaltenen Instrumenteneinheit verbunden werden. Über den Rückmeldungsanschluss kann ein Rückmeldungssignal an die Generatorsteuereinheit oder die der Schnittstelle zugeordnete Generatorsteuereinheit übertragen werden.

Das Rückmeldungssignal kann einen aktuellen Betriebsparameter des Generators und/oder eine aktuelle Anwendung des Instruments beschreiben. Das Rückmeldungssignal kann ein elektrisches Signal und/oder ein optisches Signal bzw. ein Lichtsignal sein. Beispielsweise kann das Rückmeldungssignal das Ausgangssignal beschreiben, das der Generator an das Instrument übermittelt. Das Rückmeldungssignal kann auch das aktuell behandelte Gewebe charakterisieren und/oder die aktuelle Anwendung des Instruments. Beispielsweise kann das Rückmeldungssignal eine Lichterscheinung charakterisieren, die bei der Entstehung eines Funkens an der wenigstens einen Instrumentenelektrode erzeugt wird.

Bei einem Ausführungsbeispiel hat die Schnittstelle einen Fluidanschluss, der mit einer Fluidquelle des Roboters bzw. Robotersystems verbunden ist. Über die Fluidquelle kann Fluid zu dem Fluidanschluss gefördert werden. Das Instrument einer zugeordneten Instrumenteneinheit, die an dem betreffenden Roboterarm gehalten ist, kann an den Fluidanschluss angeschlossen werden. Dadurch kann das Instrument mit Fluid versorgt werden, wie etwa einem Gas (z.B. Kohlendioxid oder Argon) oder einer Flüssigkeit (z.B. Wasser). Die Anzahl der Fluidanschlüsse und die Anzahl der bereitgestellten Fluide kann beliebig gewählt werden.

Vorteilhafte Ausgestaltungen der Erfindung ergeben sich aus den abhängigen Ansprüchen, der Beschreibung und der Zeichnung. Nachfolgend werden bevorzugte Ausführungsbeispiele der Erfindung anhand der beigefügten Zeichnung im Einzelnen erläutert. In der Zeichnung zeigen:

Figur 1 eine schematische Prinzipdarstellung eines Ausführungsbeispiels eines Robotersystems mit wenigstens einem Roboterarm, der eine Schnittstelle für wenigstens eine Instrumenteneinheit aufweist,

Figuren 2 bis 4 jeweils unterschiedliche Ausgestaltungen der Ausführung der Schnittstelle sowie der Instrumenteneinheit nach Art eines Blockschaltbildes.

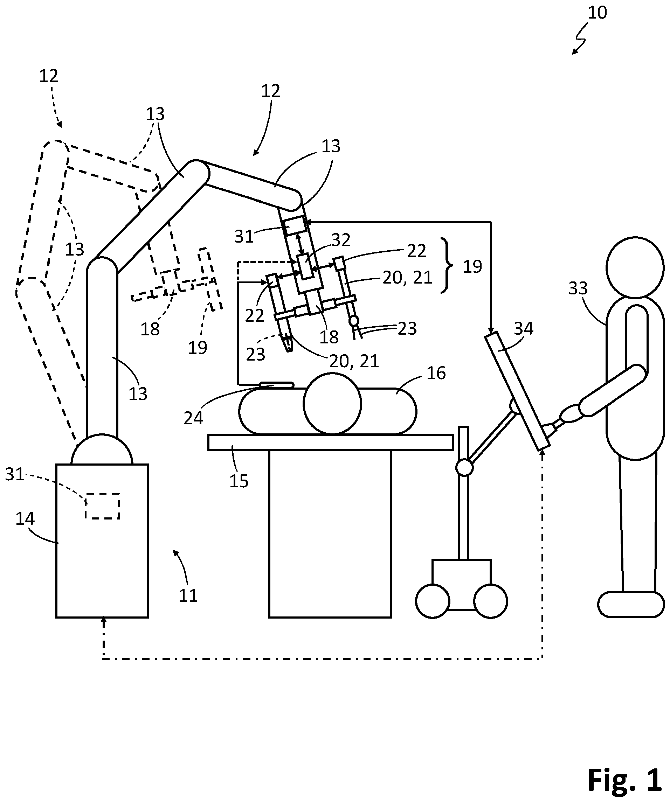

In Figur 1 ist ein Ausführungsbeispiel eines Robotersystems 10 in einer schematischen Prinzipdarstellung gezeigt. Das medizinische Robotersystem 10 hat einen Roboter 11 mit wenigstens einem Roboterarm 12. Jeder Roboterarm 12 hat mehrere beweglich miteinander verbundene Armelemente 13. Die Armelemente 13 können jeweils um eine oder mehrere unterschiedliche Achsen schwenkbar oder drehbar aneinandergelagert sein. Der Roboter 11 hat eine Basis 14, an der der wenigstens eine Roboterarm 12 angeordnet ist. Mittels der Basis 14 kann der Roboter 11 beispielsweise benachbart zu einem Behandlungstisch oder Operationstisch 15 in einem Behandlungsraum bzw. Operationssaal aufgestellt werden. Alternativ kann die Basis 14 auch am Operationstisch oder einer anderen mobilen oder immobilen Einrichtung angeordnet sein.

An einem freien Ende, das beispielsgemäß das der Basis 14 entgegengesetzte Ende des Roboterarms 12 ist, hat zumindest einer der vorhandenen Roboterarme 12 eine Halteeinrichtung 18 für eine einzige Instrumenteneinheit 19 oder für mehrere Instrumenteneinheiten 19. Die Halteeinrichtung 18 ist zur Herstellung einer formschlüssigen und/oder kraftschlüssigen mechanischen Verbindung mit der wenigstens einen Instrumenteneinheit 19 eingerichtet. Die gesamte Halteeinrichtung 18 kann mittels des betreffenden Roboterarms 12 bewegt und positioniert werden.

Die Halteeinrichtung 18 kann dazu eingerichtet sein, die wenigstens eine gehaltene Instrumenteneinheit 19 individuell zu bewegen und/oder zu positionieren, insbesondere relativ zu dem Armelement 13 des Roboterarms 12, an dem die Halteeinrichtung 18 befestigt ist. Die Halteeinrichtung 18 kann wenigstens eine Linearachse und/oder wenigstens eine rotatorische Achse aufweisen, um die wenigstens eine Instrumenteneinheit 19 zu bewegen und zu positionieren.

Ist die Halteeinrichtung 18 zum Halten von mehreren Instrumenteneinheiten 19 eingerichtet, können eine oder mehrere aktuell nicht verwendete Instrumenteneinheiten 19 in eine Ruheposition bewegt werden, in der sie keine Störkontur für die aktuell verwendete Instrumenteneinheit 19 darstellen.

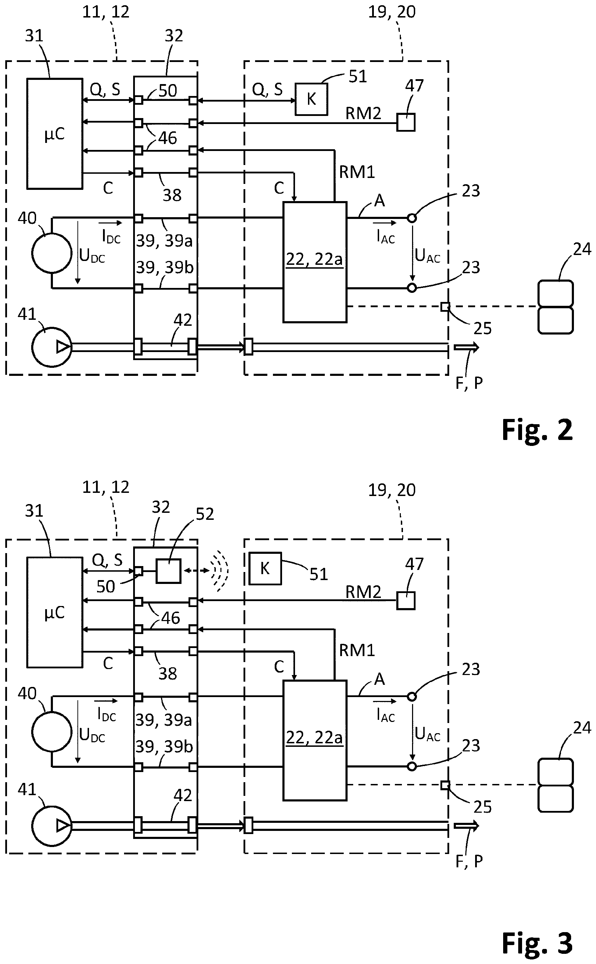

Jede Instrumenteneinheit 19 weist ein medizinisches Instrument 20, insbesondere ein elektrochirurgisches Instrument 21 sowie einen Generator 22 auf. Jeder Generator einer Instrumenteneinheit 19 ist dazu eingerichtet, für das zugeordnete Instrument 20 ein Ausgangssignal A mit einer vorgegebenen Frequenz bereitzustellen (Figuren 2 bis 4). Dazu hat der Generator 22 beispielsgemäß eine Wandlerschaltung 22a. Das Ausgangssignal A kann eine Wechselspannung U_{AC} und/oder ein Wechselstrom I_{AC} sein. Dabei kann entweder die Wechselspannung U_{AC} oder der Wechselstrom I_{AC} eingeprägt sein. Die Frequenz des Ausgangssignals A beträgt insbesondere mindestens 100 kHz und vorzugsweise höchsten 5 MHz. Die Frequenz kann beispielsweise in einem Bereich von 300 kHz bis 600 kHz liegen.

Das Ausgangssignal A des Generators 22 wird dem zugeordneten Instrument 20 derselben Instrumenteneinheit 19 bereitgestellt. Das Instrument 20 hat beispielsgemäß wenigstens eine Instrumentenelektrode 23, der das Ausgangssignal A zugeführt wird. Beispielsweise kann die Wechselspannung U_{AC} zwischen zwei Instrumentenelektroden 23 angelegt werden. Wenn über das zu behandelnde biologische Gewebe eines Patienten 16 ein Stromkreis geschlossen wird, kann über eine Instrumentenelektrode 23 ein Wechselstrom I_{AC} zum behandelten Gewebe des Patienten 16 und über eine andere Instrumentenelektrode 23 zurück zum Generator 22 fließen. Dies ist möglich, wenn das Instrument 20 als bipolares Instrument ausgeführt ist und wenigstens zwei Instrumentenelektroden 23 aufweist.

Alternativ kann das Instrument 20 als monopolares Instrument ausgeführt sein und beispielsweise nur eine einzige Instrumentenelektrode 23 aufweisen oder mehrere Instrumentenelektroden 23, die ein gemeinsames elektrisches Potenzial aufweisen. In diesem Fall kann am Patienten 16 eine Neutralelektrode 24 elektrisch leitend angebracht und über einen Neutralelektrodenanschluss 25 elektrisch mit dem Generator 22 verbunden sein, wie es optional gestrichelt in den Figuren 2 bis 4 dargestellt ist. Die Neutralelektrode 24 ist nur dann notwendig, wenn in dem Robotersystem 10 bei der Behandlung eines Patienten 16 wenigstens ein Instrument 20 verwendet wird, das als monopolares Instrument ausgebildet ist.

Der Roboter 11 weist wenigstens eine Generatorsteuereinheit 31 auf. Für jeden vorhandenen Roboterarm 12 kann bei einem Ausführungsbeispiel eine separate Generatorsteuereinheit 31 vorhanden sein. Es ist auch möglich, mehreren oder allen vorhandenen Roboterarmen 12 eine gemeinsame Generatorsteuereinheit 31 zuzuordnen. Bei einem anderen abgewandelten Ausführungsbeispiel kann für jede Instrumenteneinheit 19, die der Roboter 11 halten kann, jeweils eine individuelle Generatorsteuereinheit 31 vorhanden sein.

Die Generatorsteuereinheit 31 kann beispielsweise im oder am Roboterarm 12 angeordnet sein, wie es beispielhaft in Figur 1 gezeigt ist. Die Generatorsteuereinheit 31 kann in oder an einem Armelement 13 angeordnet sein, beispielsweise in dem Armelement 13, das die Halteeinrichtung 18 trägt. Alternativ kann die wenigstens eine Generatorsteuereinheit 31 in einem anderen Armelement 13 oder in der Basis 14 angeordnet werden, wie es beispielhaft gestrichelt in Figur 1 angedeutet ist.

Die Generatorsteuereinheit 31 ist mit einer Schnittstelle 32 zumindest eines Roboterarms 12 kommunikationsverbunden. Diese Verbindung ist vorzugsweise eine elektrische Verbindung, kann aber zusätzlich oder alternativ auch eine optische Verbindung sein. Vorzugsweise ist zumindest die Verbindung zwischen der Generatorsteuereinheit 31 und der Schnittstelle 32 über wenigstens eine elektrische und/oder optische Leitung realisiert.

Jeder Roboterarm 12 hat eine Schnittstelle 32. Die Schnittstelle 32 ist vorzugsweise benachbart zur Halteeinrichtung 18 angeordnet und befindet sich beim Ausführungsbeispiel in bzw. an dem Armelement 13, an dem auch die Halteeinrichtung 18 für die wenigstens eine Instrumenteneinheit 19 angeordnet ist. Mittels der Schnittstelle 32 kann die an dem betreffenden Roboterarm 12 gehaltene wenigstens eine Instrumenteneinheit 19 mit der Generatorsteuereinheit 31 für diesen Roboterarm 12 verbunden werden, insbesondere über eine elektrische und/oder optische Verbindung. Grundsätzlich kann die elektrische und/oder optische Verbindung drahtlos und/oder drahtgebunden sein.

Wie es in Figur 1 gezeigt ist, kann eine Bedienperson 33 (z.B. ein Operateur) mittels einer Bedieneinrichtung 34 den Roboter 11 steuern. Beispielsweise kann die Bedienperson 33 mittels der Bedieneinrichtung 34 Störbefehle an die wenigstens eine Generatorsteuereinheit übermitteln, um den Betrieb der wenigstens einen Instrumenteneinheit 19 zu steuern. Mittels der Bedieneinrichtung 34 können auch die Stelleinrichtungen des Roboters 11, insbesondere des wenigstens einen Roboterarms 12 und der wenigstens einen Halteeinrichtung 18 gesteuert werden, um das wenigstens eine medizinische Instrument 20 zu bewegen und/oder zu positionieren, was in Figur 1 schematisch durch den strichpunktiert gezeigten Doppelpfeil dargestellt ist. Die Bedieneinrichtung 34 kann hierfür die erforderlichen Bedienelemente aufweisen. Die Bedieneinrichtung 34 kann auch Ausgabemittel (z.B. akustische und/oder optische Ausgabemittel) aufweisen, um der Bedienperson 33 Informationen über die aktuelle Anwendung des Roboters 11 bzw. des wenigstens einen medizinischen Instruments 20 bereitzustellen. Beispielsweise kann die Bedieneinrichtung 34 einen Bildschirm, insbesondere einen berührungsempfindlichen Bildschirm als Schnittstelle aufweisen.

Die verschiedenen Ausgestaltungsmöglichkeiten der Instrumenteneinheit 19, des Generators 22 sowie der Schnittstelle 32 sind anhand von Ausführungsbeispielen in den Blockschaltbildern der Figuren 2 bis 4 dargestellt.

Die Schnittstelle 32 des Roboterarms 12 hat wenigstens einen Steueranschluss 38. Der Steueranschluss 38 ist mit der Generatorsteuereinheit 31 kommunikationsverbunden, so dass ein Generatorsteuersignal C an den Steueranschluss 38 und von dort weiter an einen Generator 22 einer Instrumenteneinheit 19 übertragen werden kann, die an den Steueranschluss 38 angeschlossen ist. Über die Generatorsteuereinheit 31 wird mithin der Betrieb des Generators 22 gesteuert.

Das hochfrequente Ausgangssignal A wird somit außerhalb des Roboterarms 12 in der Instrumenteneinheit 19 durch den Generator 22 erzeugt und dem Instrument 20 der jeweiligen Instrumenteneinheit 19 bereitgestellt (z.B. hochfrequenter Wechselstrom I_{AC} und/oder hochfrequente Wechselspannung U_{AC}). Über das Generatorsteuersignal C können die Parameter für das Ausgangssignal A vorgegeben und eingestellt werden, beispielsweise eine Frequenz und/oder eine Amplitude und/oder ein Scheitelfaktor ("crest factor") und/oder eine Wellenform ("waveform") und/oder ein Tastgrad ("duty cycle") im Falle eines pulsweiten modulierten Signals, usw. Dadurch lässt sich der Betrieb des Instruments 20 geeignet steuern, beispielsweise zum Koagulieren, Fusionieren oder Schneiden von biologischem Gewebe eines bestimmten Gewebetyps.

Damit der Generator 22 das Ausgangssignal A für das Instrument 20 erzeugen kann, kann die Schnittstelle wenigstens einen Energieversorgungsanschluss 39 aufweisen. Beim Ausführungsbeispiel hat die Schnittstelle 32 einen ersten Energieversorgungsanschluss 39a sowie einen zweiten Energieversorgungsanschluss 39b. An die beiden Energieversorgungsanschlüsse 39a, 39b sind die zwei Pole einer Energiequelle 40 angeschlossen. Die Energiequelle 40 kann zum Beispiel eine Gleichspannungsquelle oder eine Gleichstromquelle zur Bereitstellung einer eingeprägten Gleichspannung U_{DC} oder eines eingeprägten Gleichstromes I_{DC} sein. Über die Energieversorgungsanschlüsse 39a, 39b wird die elektrische Energie an den Generator 22 einer an die Energieversorgungsanschlüsse 39a, 39b angeschlossenen Instrumenteneinheit 19 übertragen. Unter Einsatz der bereitgestellten elektrischen Energie kann der Generator 22 (bzw. die Wandlerschaltung 22a des Generators 22) das Ausgangssignal A erzeugen.

Somit kann die Schnittstelle 32 auch zur Energie- bzw. Leistungsversorgung des Generators 22 dienen. Alternativ dazu könnte die Instrumenteneinheit 19 auch ohne Zwischenschaltung der Schnittstelle 32 mit elektrischer Energie versorgt werden.

Die Schnittstelle 32 kann optional einen mit einer Fluidquelle 41 fluidisch verbundenen oder verbindbaren Fluidanschluss 42 aufweisen. Mittels des Fluidanschlusses 42 kann ein Instrument 20 einer daran angeschlossenen Instrumenteneinheit 19 mit wenigstens einem Fluid versorgt werden, beispielsweise einem Gas und/oder einer Flüssigkeit, sofern dies für den Betrieb des betreffenden Instruments 20 erforderlich oder vorteilhaft ist. Beispielsweise kann mittels des Fluidanschlusses 42 der Schnittstelle 32 ein als Plasmainstrument ausgebildetes Instrument 20 mit einem geeigneten Gas (z.B. Argon) versorgt werden. Mittels der wenigstens einen Instrumentenelektrode 23 kann ein Plasma gezündet bzw. erzeugt werden und als Plasmastrom P aus dem Instrument 20 ausgestoßen werden. Dem Instrument 20 können auch andere Fluide, beispielsweise Wasser oder eine andere Flüssigkeit, zugeführt werden, um einen entsprechenden Fluidstrom F zu erzeugen und auszustoßen.

Bei den in den Figuren 2 bis 4 veranschaulichten Ausführungsbeispielen hat die Schnittstelle 32 außerdem wenigstens einen Rückmeldungsanschluss 46. Der wenigstens eine Rückmeldungsanschluss 46 ist mit der betreffenden Generatorsteuereinheit 31 kommunikationsverbunden. Über jeden Rückmeldungsanschluss 46 kann von einer daran angeschlossenen Instrumenteneinheit 19 ein Rückmeldungssignal RMi (i = 1, 2, ..., n) übertragen werden. Lediglich beispielhaft sind in den Figuren 2 bis 4 jeweils zwei Rückmeldungsanschlüsse 46 zur Übertragung eines ersten Rückmeldungssignals RM1 und eines zweiten Rückmeldungssignals RM2 veranschaulicht. Bei dem wenigstens einen Rückmeldungssignal RM kann es sich um ein elektrisches und/oder optisches Signal handeln.

Das erste Rückmeldungssignal RM1 kann beispielsweise einen aktuellen Betriebsparameter des Generators 22 charakterisieren, beispielsweise einen Wechselstrom I_{AC}, der sich aufgrund einer als Ausgangssignal A eingeprägten Wechselspannung U_{AC} ergibt oder umgekehrt. Das zweite Rückmeldungssignal RM2 kann beispielsweise eine Funkenbildung an der wenigstens einen Instrumentenelektrode 23 charakterisieren, die mit einem geeigneten Sensor 47 erfasst wurde.

Bei den erfindungsgemäßen Ausführungsbeispielen kann optional eine automatische Erkennung eines Instruments 20 bzw. eines Instrumententyps erfolgen. Dadurch kann beispielsweise in der Generatorsteuereinheit 31 automatisch erkannt werden, was für ein Instrumententyp in einer an die Schnittstelle 32 angeschlossenen Instrumenteneinheit 19 eingesetzt wird. Beispielsweise können Instrumententypen voneinander unterschieden werden, wie Plasmainstrumente (z.B. Plasmakoagulationsinstrumente), elektrochirurgische Schneidinstrumente, elektrochirurgische Koagulationsinstrumente mit zwei oder mehr Koagulationselektroden, Thermofusionsinstrumente, usw. Zusätzlich kann auch festgestellt werden, ob es sich bei dem jeweiligen Instrument 20 um ein monopolares oder bipolares Instrument handelt. Allgemein können die Art bzw. der Typ eines Instruments 20 durch eine oder mehrere der nachfolgend genannten Eigenschaften in beliebiger Kombination gekennzeichnet sein:
- die Anzahl der vorhandenen Elektroden,
- die Ausführung als monopolares oder bipolares Instrument,
- ein Instrument, das ein Fluid (Gas und/oder Flüssigkeit) zur Gewebebeeinflussung benötigt und/oder verwendet,
- die wenigstens eine Anwendung des Instruments (Schneiden, Koagulieren, Fusionieren, etc.).

Zur Identifikation eines Instruments 20 kann eine Kennung K über die Schnittstelle 32 an die Generatorsteuereinheit 31 übermittelt werden. Hierzu weist die Schnittstelle 32 beim Ausführungsbeispiel einen Identifikationsanschluss 50 auf, mittels dem ein Identifikationssignal S an die Generatorsteuereinheit 31 übermittelt werden kann. Beispielsweise kann die Kennung K von der Generatorsteuereinheit 31 drahtlos oder drahtgebunden von einem Datenträger 51 der Instrumenteneinheit 19 ausgelesen und in Form des Identifikationssignals S an die Generatorsteuereinheit 31 übertragen werden. Der Datenträger 51 kann Bestandteil des Generators 22 oder des Instruments 20 sein.

Das Auslesen der Kennung K kann durch die Generatorsteuereinheit 31 veranlasst werden, beispielsweise indem die Generatorsteuereinheit 31 über den Identifikationsanschluss 50 ein Anfragesignal Q bzw. Auslesesignal an den Datenträger 51 übermittelt (Figur 2). Bei dem in Figur 2 dargestellten Ausführungsbeispiel ist die Verbindung zwischen dem Identifikationsanschluss 50 und dem Datenträger 51 drahtgebunden realisiert.

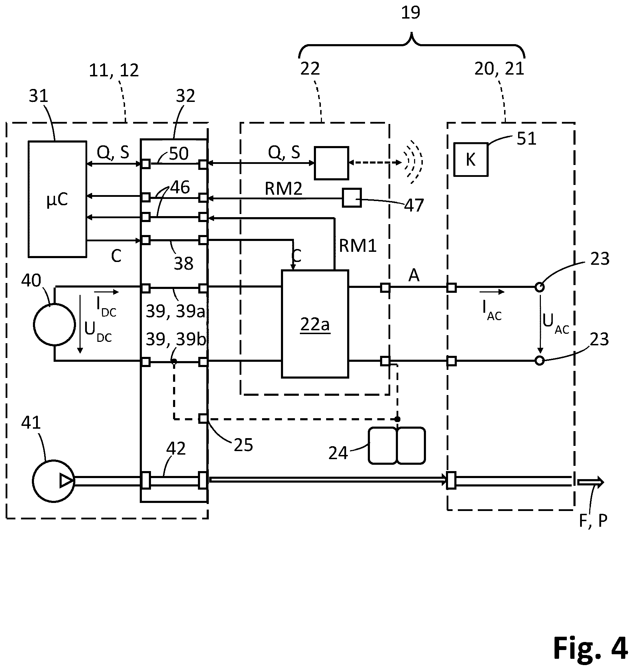

Das Auslesen der Kennung K von einem geeigneten Datenträger 51 kann in Abwandlung zum Ausführungsbeispiel gemäß Figur 2 auch mittels einer Erfassungseinheit 52 ausgeführt werden, die insbesondere außerhalb des Instruments 20 angeordnet ist, beispielsweise im Roboterarm 12 bzw. in der Schnittstelle 32 des Roboterarms 12 (Figur 3) oder Bestandteil des Generators 22 ist (Figur 4). Die Erfassungseinheit 52 kann beispielsweise die Kennung K mittels Nahfeldkommunikation (NFC) bzw. mittels einer RFID-Verbindung vom Datenträger 51 auslesen und ein die Kennung K beschreibendes Identifikationssignal S an die Generatorsteuereinheit 31 übermitteln. Das Auslesen der Kennung K mittels der Erfassungseinheit 52 kann durch das Anfragesignal Q ausgelöst werden, das von der Generatorsteuereinheit 31 an die Erfassungseinheit 52 über den Identifikationsanschluss 50 übermittelt wird (Figuren 3 und 4).

Bei dem in Figuren 2 und 3 schematisch dargestellten Ausführungsbeispielen sind der Generator 22 und das Instrument 20 als gemeinsame integrale Komponente ausgeführt und insbesondere in einem gemeinsamen Gehäuse angeordnet. In Abwandlung hierzu können der Generator 22 und das Instrument 20 auch durch separate Komponenten realisiert sein, die mechanisch verbunden und kommunikationsverbunden sind (Figur 4). Beispielsweise kann der Generator 22 und das Instrument 20 in jeweils einem separaten Gehäuseteil realisiert werden, wobei die beiden Gehäuseteile mechanisch lösbar oder unlösbar miteinander verbunden werden und dabei auch eine Kommunikationsverbindung, beispielsweise elektrische und/oder optische Verbindung hergestellt wird.

Bei den in den Figuren 2 bis 4 veranschaulichten Ausführungsbeispielen kann ein optional vorhandener Neutralelektrodenanschluss 25 zum Anschluss einer Neutralelektrode 24 am Generator 22 vorhanden sein. Zusätzlich oder alternativ kann der Neutralelektrodenanschluss 25 auch Bestandteil der Schnittstelle 32 am Roboterarm 12 sein (gestrichelter Pfeil in Figur 1). Wenn beispielsweise das Instrument 20 als monopolares Instrument ausgebildet ist, so dass für die wenigstens eine Instrumentenelektrode 23 ein einziger Energieversorgungsanschluss 39 (z.B. erster Energieversorgungsanschluss 39a) ausreicht, kann ein weiterer Energieversorgungsanschluss (z.B. zweiter Energieversorgungsanschluss 39b) als Neutralelektrodenanschluss 25 verwendet werden. Diese Möglichkeit ist auch schematisch gestrichelt in Figur 4 veranschaulicht.

Die Erfindung betrifft ein medizinisches Robotersystem 10 mit einem wenigstens einen Roboterarm 12 aufweisenden Roboter 11. An dem wenigstens einen Roboterarm 12 ist wenigstens eine Instrumenteneinheit 19 gehalten und mittels des Roboterarms 12 bewegbar und/oder positionierbar. Jede Instrumenteneinheit 19 hat ein medizinisches Instrument 20, vorzugsweise elektrochirurgisches Instrument 21, sowie einen Generator 22, der ein Ausgangssignal A, insbesondere HF-Ausgangssignal, für das Instrument 20 erzeugt und bereitstellt. Am Roboterarm 12 ist eine Schnittstelle 32 angeordnet, mit der jede am Roboterarm 12 gehaltene Instrumenteneinheit 19 zur Herstellung einer Kommunikationsverbindung elektrisch und/oder optisch gekoppelt werden kann. Die Verbindung kann drahtlos und/oder drahtgebunden sein. Der Roboter 11 bzw. der Roboterarm 12 hat wenigstens eine Generatorsteuereinheit 31, wobei jede Schnittstelle 32 mit einer zugeordneten Generatorsteuereinheit 31 kommunikationsverbunden ist, um ein Generatorsteuersignal C von der Generatorsteuereinheit 31 an die wenigstens eine an die Schnittstelle 32 angeschlossene Instrumenteneinheit 19 zu übertragen. Der Generator 22 und die Generatorsteuereinheit 31 sind somit voneinander getrennt, wobei sich der Generator 22 außerhalb des Roboters 11 befindet, während die wenigstens eine Generatorsteuereinheit 31 Bestandteil des Roboters 11 ist und in den Roboter 11 bzw. den Roboterarm 12 integriert sein kann.

### Bezugszeichenliste:

- 10: medizinisches Robotersystem
- 11: Roboter
- 12: Roboterarm
- 13: Armelement
- 14: Basis
- 15: Operationstisch
- 16: Patient

- 18: Halteeinrichtung
- 19: Instrumenteneinheit
- 20: medizinisches Instrument
- 21: elektrochirurgisches Instrument
- 22: Generator
- 23: Instrumentenelektrode
- 24: Neutralelektrode
- 25: Neutralelektrodenanschluss

- 31: Generatorsteuereinheit
- 32: Schnittstelle
- 33: Bedienperson
- 34: Bedieneinrichtung

- 38: Steueranschluss
- 39: Energieversorgungsanschluss
- 39a: erster Energieversorgungsanschluss
- 39b: zweiter Energieversorgungsanschluss
- 40: Energiequelle
- 41: Fluidquelle
- 42: Fluidanschluss
- 46: Rückmeldungsanschluss
- 47: Sensor

- 50: Identifikationsanschluss
- 51: Datenträger
- 52: Erfassungseinheit

- A: Ausgangssignal
- C: Generatorsteuersignal
- F: Fluidstrom
- I_{AC}: Wechselstrom
- I_{DC}: Gleichstrom
- K: Kennung
- P: Plasmastrom
- RMi: Rückmeldungssignal
- RM1: erstes Rückmeldungssignal
- RM2: zweites Rückmeldungssignal
- Q: Anfragesignal
- S: Identifikationssignal
- U_{AC}: Wechselspannung
- U_{DC}: Gleichspannung

## Patentansprüche

1. Medizinisches Robotersystem (10), aufweisend:
- wenigstens eine Instrumenteneinheit (19) jeweils aufweisend ein medizinisches Instrument (20), insbesondere ein elektrochirurgisches Instrument (21), und einen diesem Instrument (20) zugeordneten Generator (22), der mit dem Instrument (20) verbunden und dazu eingerichtet ist, ein mit einer Frequenz alternierendes elektrisches Ausgangssignal (A) für das zugeordnete Instrument (20) bereitzustellen,
- einen Roboter (11) mit wenigstens einer Generatorsteuereinheit (31) und mit wenigstens einem Roboterarm (12), der dazu eingerichtet ist, die Instrumenteneinheit (19) oder wenigstens eine der vorhandenen Instrumenteneinheiten (19) zu halten,
- eine am Roboterarm (12) angeordnete Schnittstelle (32), die mit der diesem Roboterarm (12) zugeordneten Generatorsteuereinheit (31) verbunden ist und die wenigstens einen Steueranschluss (38) aufweist, an den jeweils ein Generator (22) einer an diesem Roboterarm (12) gehaltenen Instrumenteneinheit (19) anschließbar ist, um ein Generatorsteuersignal (C) von der Generatorsteuereinheit (31) an den Generator (22) zu übertragen.

2. Medizinisches Robotersystem nach Anspruch 1, wobei bei der Instrumenteneinheit (19) oder bei wenigstens einer der vorhandenen Instrumenteneinheiten (19) der Generator (22) lösbar oder unlösbar am Instrument (20) befestigt ist.

3. Medizinisches Robotersystem nach Anspruch 1 oder 2, wobei bei der Instrumenteneinheit (19) oder bei wenigstens einer der vorhandenen Instrumenteneinheiten (19) der Generator (22) im Instrument (20) angeordnet ist.

4. Medizinisches Robotersystem nach einem der vorhergehenden Ansprüche, wobei das Instrument (20) wenigstens eine Instrumentenelektrode (23) aufweist, die mit dem Generator (22) der Instrumenteneinheit (19) elektrisch verbunden ist.

5. Medizinisches Robotersystem nach einem der vorhergehenden Ansprüche, wobei der Generator (22) der Instrumenteneinheit (19) oder wenigstens einer der vorhandenen Instrumenteneinheiten (19) und/oder an der Schnittstelle (32) einen Neutralelektrodenanschluss (25) für eine Neutralelektrode (24) aufweist.

6. Medizinisches Robotersystem nach einem der vorhergehenden Ansprüche, wobei die Schnittstelle (32) wenigstens einen Identifikationsanschluss (50) aufweist, mit dem jeweils ein Generator (22) einer an diesem Roboterarm (12) gehaltenen Instrumenteneinheit (19) verbindbar ist, um ein Identifikationssignal (S) an die zugeordnete Generatorsteuereinheit (31) zu übertragen.

7. Medizinisches Robotersystem nach Anspruch 6, wobei der Generator (22) und/oder die Schnittstelle (32) eine Erfassungseinheit (52) aufweist, die mit dem Identifikationsanschluss (50) verbindbar ist und die dazu eingerichtet ist, eine Kennung (K) des zugeordneten Instruments (20) zu erfassen.

8. Medizinisches Robotersystem nach einem der vorhergehenden Ansprüche, wobei die Schnittstelle (32) wenigstens einen Energieversorgungsanschluss (39) aufweist, der mit einer Energiequelle (41) verbunden ist und an den jeweils ein Generator (22) einer an diesem Roboterarm (12) gehaltenen Instrumenteneinheit (19) anschließbar ist, um den Generator (22) mit elektrischer Energie zu versorgen.

9. Medizinisches Robotersystem nach Anspruch 8, wobei mittels der Energiequelle (40) an dem Energieversorgungsanschluss (39) eine Gleichspannung (U_{DC}) und/oder ein Gleichstrom (I_{DC}) bereitgestellt wird.

10. Medizinisches Robotersystem nach einem der vorhergehenden Ansprüche, wobei die Schnittstelle (32) wenigstens einen Rückmeldungsanschluss (46) aufweist, mit dem jeweils ein Generator (22) einer an diesem Roboterarm (12) gehaltenen Instrumenteneinheit (19) verbindbar ist, um wenigstens ein Rückmeldungssignal (RM) an die zugeordnete Generatorsteuereinheit (31) zu übertragen.

11. Medizinisches Robotersystem nach Anspruch 10, wobei das wenigstens eine Rückmeldungssignal (RM) einen aktuellen Betriebsparameter des Generators (22) und/oder eine aktuelle Anwendung des Instruments (20) beschreibt.

12. Medizinisches Robotersystem nach einem der vorhergehenden Ansprüche, wobei die Schnittstelle (32) wenigstens einen Fluidanschluss (42) aufweist, der mit einer Fluidquelle (41) verbunden ist und an den jeweils ein Instrument (20) einer an diesem Roboterarm (12) gehaltenen Instrumenteneinheit (19) anschließbar ist, um das Instrument (20) mit dem Fluid zu versorgen.

## Geänderte Patentansprüche

### Geänderte Patentansprüche gemäss Regel 137(2) EPÜ.

1. Medizinisches Robotersystem (10), aufweisend:
- wenigstens eine Instrumenteneinheit (19) jeweils aufweisend ein elektrochirurgisches (21) mit wenigstens einer Instrumentenelektrode (23), und einen diesem elektrochirurgischen Instrument (21) zugeordneten Generator (22), der mit dem elektrochirurgischen Instrument (21) verbunden und dazu eingerichtet ist, der Instrumentenelektrode (23) des zugeordneten elektrochirurgischen Instruments (21) ein mit einer Frequenz alternierendes elektrisches Ausgangssignal (A) bereitzustellen, wobei der Generator (22) Bestandteil des elektrochirurgischen Instruments (21) ist oder ein Gehäuseteil hat, das lösbar oder unlösbar mechanisch mit dem Gehäuseteil des elektrochirurgischen Instruments (21) verbunden ist, wodurch eine Kommunikationsverbindung zwischen dem Generator (22) und dem elektrochirurgischen Instrument (21) hergestellt ist.
- einen Roboter (11) mit wenigstens einer Generatorsteuereinheit (31) und mit wenigstens einem Roboterarm (12), der dazu eingerichtet ist, die Instrumenteneinheit (19) oder wenigstens eine der vorhandenen Instrumenteneinheiten (19) zu halten,
- eine am Roboterarm (12) angeordnete Schnittstelle (32), die mit der diesem Roboterarm (12) zugeordneten Generatorsteuereinheit (31) verbunden ist und die wenigstens einen Steueranschluss (38) aufweist, an den jeweils ein Generator (22) einer an diesem Roboterarm (12) gehaltenen Instrumenteneinheit (19) anschließbar ist, um ein Generatorsteuersignal (C) von der Generatorsteuereinheit (31) an den Generator (22) zu übertragen.

2. Medizinisches Robotersystem nach Anspruch 1, wobei bei der Instrumenteneinheit (19) oder bei wenigstens einer der vorhandenen Instrumenteneinheiten (19) der Generator (22) im Instrument (20) angeordnet ist.

3. Medizinisches Robotersystem nach einem der vorhergehenden Ansprüche, wobei der Generator (22) der Instrumenteneinheit (19) oder wenigstens einer der vorhandenen Instrumenteneinheiten (19) und/oder an der Schnittstelle (32) einen Neutralelektrodenanschluss (25) für eine Neutralelektrode (24) aufweist.

4. Medizinisches Robotersystem nach einem der vorhergehenden Ansprüche, wobei die Schnittstelle (32) wenigstens einen Identifikationsanschluss (50) aufweist, mit dem jeweils ein Generator (22) einer an diesem Roboterarm (12) gehaltenen Instrumenteneinheit (19) verbindbar ist, um ein Identifikationssignal (S) an die zugeordnete Generatorsteuereinheit (31) zu übertragen.

5. Medizinisches Robotersystem nach Anspruch 4, wobei der Generator (22) und/oder die Schnittstelle (32) eine Erfassungseinheit (52) aufweist, die mit dem Identifikationsanschluss (50) verbindbar ist und die dazu eingerichtet ist, eine Kennung (K) des zugeordneten Instruments (20) zu erfassen.

6. Medizinisches Robotersystem nach Anspruch 5, wobei die Erfassungseinheit (52) dazu eingerichtet ist, die Kennung (K) drahtlos elektromagnetisch und/oder optisch zu erfassen.

7. Medizinisches Robotersystem nach einem der Ansprüche 4 bis 6, wobei das Identifikationssignal (S) eine Zuordnung des elektrochirurgischen Instruments (21) der an diesem Roboterarm (12) gehaltenen Instrumenteneinheit (19) zu einem Instrumententyp der folgenden Instrumententypen ermöglicht: Plasmainstrument, elektrochirurgisches Schneidinstrument, elektrochirurgisches Koagulationsinstrument mit zwei oder mehr Koagulationselektroden, Thermofusionsinstrument.

8. Medizinisches Robotersystem nach einem der vorhergehenden Ansprüche, wobei die Schnittstelle (32) wenigstens einen Energieversorgungsanschluss (39) aufweist, der mit einer Energiequelle (41) verbunden ist und an den jeweils ein Generator (22) einer an diesem Roboterarm (12) gehaltenen Instrumenteneinheit (19) anschließbar ist, um den Generator (22) mit elektrischer Energie zu versorgen.

9. Medizinisches Robotersystem nach Anspruch 8, wobei mittels der Energiequelle (40) an dem Energieversorgungsanschluss (39) eine Gleichspannung (U_{DC}) und/oder ein Gleichstrom (I_{DC}) bereitgestellt wird.

10. Medizinisches Robotersystem nach einem der vorhergehenden Ansprüche, wobei die Schnittstelle (32) wenigstens einen Rückmeldungsanschluss (46) aufweist, mit dem jeweils ein Generator (22) einer an diesem Roboterarm (12) gehaltenen Instrumenteneinheit (19) verbindbar ist, um wenigstens ein Rückmeldungssignal (RM) an die zugeordnete Generatorsteuereinheit (31) zu übertragen.

11. Medizinisches Robotersystem nach Anspruch 10, wobei das wenigstens eine Rückmeldungssignal (RM) einen aktuellen Betriebsparameter des Generators (22) und/oder eine aktuelle Anwendung des Instruments (20) beschreibt.

12. Medizinisches Robotersystem nach einem der vorhergehenden Ansprüche, wobei die Schnittstelle (32) wenigstens einen Fluidanschluss (42) aufweist, der mit einer Fluidquelle (41) verbunden ist und an den jeweils ein Instrument (20) einer an diesem Roboterarm (12) gehaltenen Instrumenteneinheit (19) anschließbar ist, um das Instrument (20) mit dem Fluid zu versorgen.
